# EUROPEAN PATENT SPECIFICATION

(11) **EP 1 713 348 B1**
(45) Date of publication and mention of the grant of the patent: **28.05.2008**
(21) Application number: 05701227.0
(22) Date of filing: 28.01.2005
(51) Int. Cl.: A23K 1/16, A23K 1/00, C12N 9/14

(54) **GRANULES CONTAINING STABILIZED PHYTASE FORMULATIONS**
STABILISIERTE PHYTASE ENTHALTENDE GRANULATE
GRANULES CONTENANT DES PHYTASES STABILISEES

(30) Priority: 30.01.2004 EP 04002056
(43) Date of publication of application: 25.10.2006
(73) Proprietor: BASF SE, 67056 Ludwigshafen (DE)
(72) Inventor: HABICH, Andreas, 67346 Speyer (DE); BRAUN, Jörg, 76879 Essingen (DE); LOHSCHEIDT, Markus, 69121 Heidelberg (DE)
(86) International application number: PCT/EP2005/000827
(87) International publication number: WO 2005/074707

(56) References cited:
- EP-A- 0 257 996
- EP-A- 0 897 985
- WO-A-98/28408
- WO-A-98/54980
- WO-A-03/040398
- US-A1- 2002 034 798
- US-B1- 6 610 519
- MARRS W M: "RHEOLOGY AS AN INDICATOR OF FUNCTIONALITY" WORLD OF INGREDIENTS, C&S PUBLISHERS, ARNHEM, NL, February 1996 (1996-02), pages 38-40, XP009028458 ISSN: 1380-491X

## Description

### Field of the Invention

The present invention relates to solid or liquid phosphatase formulations having an increased stability, preferably thermo stability, which is obtained by the addition of stabilizing agents

The present invention relates to the formulation of phytases, into carbohydrate (e.g. starch-) containing granulates and/or liquid formulations, and relates to processes for the preparation of such enzyme-containing granulates and liquid formulations. These (edible) granulates and liquid formulations can then be used in animal feed and/or human nutrition. The enzyme containing granulates and the liquid formulations display an improved storage and processing stability.

### Background of the Invention

The use of phosphatases in human nutrition or animal, e.g. livestock, feed has become almost common practice. These enzymes are usually produced by culturing micro organisms in large scale fermenters operated by industrial enzyme producers. At the end of the fermentation the resulting "broth" is usually subjected to a series of filtration steps to separate the biomass (the micro organisms) from the desired enzyme (in-solution). The enzyme solution is either then sold as a liquid or processed to a dry formulation.

Enzyme liquid and dry formulations comprising phosphatases, preferably phytases, are used on a commercial scale by the feed and food industry. Liquid formulations may be added to the feed or food after pelleting in order to avoid heat inactivation of the enzyme(s) which would occur during the pelleting process. However the amounts of enzyme in the final feed or food preparations are usually very small which makes it difficult to achieve a homogenous distribution of the enzyme in the feed or food, and liquids are notoriously more difficult to mix evenly than dry ingredients. In addition one needs specialised (expensive) equipment to add liquids to the feed after pelleting which is not currently available at most feed mills (due to the extra cost). Even when applying liquid formulations comprising enzymes, the storage stability of such formulations often is a problem.

Dry formulations of phosphatases, especially phytases, on the other hand, have the disadvantage of heat-inactivation of the enzymes during pelleting. Preferred manufacturing protocols in the feed and food industry involve steam pelleting where the feed or food is subjected to steam injection(s) prior to pelleting. In the subsequent pelleting step the feed or food is forced through a matrix or die and the resulting strips are cut into suitable pellets of variable length. The moisture content immediately before pelleting is generally between 10% and 20%. During this process temperatures may rise to 60-95°C. The combined effect of high moisture content and high temperature is detrimental to most enzymes. These disadvantages are also encountered in other types of thermo mechanical treatments such as extrusion and expansion.

Various enzyme manufacturers have developed alternative formulation methods to try to improve the stability of dry and liquid enzyme products during pelleting and storage.

EP 0 758 018 A1 discloses a method to improve the processing and storage stability of dry enzyme preparations by adding inorganic salts.

GB 2167 758 discloses an enzyme granulating method and a granular composition containing an enzyme comprising 1 to 35% by weight of an enzyme and from 0.5 to 30% by weight of a synthetic fibre chip or pulp.

EP 1 069 832 A1 (WO 00/47060) describes a process for the preparation of enzyme containing granulates where an aqueous enzyme-containing liquid is mixed with a solid carrier and optionally additive ingredients and is mechanically processed into granules, dried and subsequently coated with polyethylene glycol.

EP 858 266 (WO 97/16076) discloses a particulate, enzyme-containing preparation suitable for the use in the manufacture of animal feed compositions.

EP 862 623 A1 (WO 97/12958) discloses a microgranular enzyme composition having an average particle size of from about 20 to 400 microns.

None of the cited references discloses phosphatase-containing formulations which are stabilized with the gums specified herein or animal proteins.

DE 1 958 104 describes a process for the preparation of enzyme containing granulates suitable for detergent and laundry compositions wherein alkali salts of inorganic or organic acid are used as carrier.

DE 2137 043 and DE 2 137 042 disclose an enzyme containing composition suitable for detergent compositions wherein the carrier is comprised of an inorganic salt.

EP 716 685 discloses multiple enzyme granulates suitable for incorporation in detergents and cleaning compositions containing an enzyme and inorganic and/or organic carrier material.

WO 00/36927 discloses a method for producing granulates which contain enzymes and which are suited for feeding animals.

WO 03/059087 describes a process for the preparation of an enzyme-containing granulate wherein an aqueous-containing liquid, optionally supplemented with a solid carrier and/or additive ingredients, is processed into granules, dried and subsequently coated with a polyolefin.

WO 03/059086 describes a process for the preparation of an enzyme-containing granulate wherein an aqueous-containing liquid, optionally supplemented with a solid carrier and/or additive ingredients, is processed into granules, dried and subsequently coated with a dispersion containing particle of a hydrophobic substance, preferably a polyolefin.

For feed application a stable, preferably thermostable, phosphatase is of general inter est in order to avoid problems that may occur during the formulation (e.g. spray drying, granulation) and feed treatment processes (e.g. pelleting, extrusion, expansion) where temporarily high temperatures (up to 80-120°C), moisture and shear stress may affect the protein structure and lead to an undesired loss of activity.

Phosphatases are generally added to feed and food preparations for various reasons. In food applications phosphatases are added for example in baking or brewery. The function of phosphatases, especially phytases in feed application is often to improve the feed conversion rate, e.g. by reducing the digesta-viscosity or by reducing the anti-nutritional effect of certain feed compounds. Phosphatases, preferably phytases can also be used, such as to reduce the amount of compounds in the manure which are harmful to the environment.

In all the various applications, these enzymes are often exposed to thermal challenge, e.g. heat, moisture or temperature exposure, which can lead to a partial or complete inactivation of the enzyme.

Although a large amount of phosphate is present in feed in form of phytate phosphorus, monogastric animals, like pigs and poultry, lack the ability to use this form of phosphate. The alkali or earth alkali salts of phytic acid occur naturally mainly in cereals. Since monogastric animals are not able to use this form of phosphate it is common practice to add inorganic phosphates to animal feed.

On the other hand an enzyme called phytase (myo-inositol hexakisphosphate phosphohydrolase) is known to occur in plants and in some micro organisms. Since phytase can be produced by fermentation it is known in the art to use phytase as an animal feed additive in order to enhance the nutritive value of plant material by liberation of inorganic phosphate from phytic acid (myo-inositol hexakisphosphate). By adding phytase to the animal feed the level of phosphorus pollution of the environment can be reduced since the animal is able to make use of the phosphate liberated from phytate by the use of phytase.

The international patent application WO 93/16175 (EP 626 010) of Gist-Brocades describes stabilized liquid formulations of phytase. It is suggested to use as stabilizing agent urea and a water-soluble polyol whereby sorbitol, glycerol and polyethylene glycol having a molecular weight of 6000 are mentioned.

The European patent application EP-A1 0 969 089 of Hoffmann-La Roche describes stabilized enzyme formulation comprising phytase and at least one stabilizing agent selected from the group consisting of a) polyols containing five carbon atoms, preferably C5 sugars, more preferably xylitol or ribitol, b) polyethylene glycol having a molecular weight of 600 to 4000 Da, c) the disodium salts of malonic, glutaric and succinic acid, d) carboxymethylcellulose, and e) sodium alginate. It furthermore describes stabilizing phytase formulation by cross-linking either by chemical reactions with glutaraldehyde; or by b) oxidation with sodium periodate and subsequent addition of adipic acid dihydrazide.

WO 98/54980 describes phytase containing granules and WO 98155599 describe high-activity phytase liquids and feed preparations containing them.

Despite the different approaches in the state of the art, there is still a need for stabilized phosphatase formulations that are suitable for human and/or animal nutrition and that display an enhanced stability during processing, pelleting and storage. The formulations should be easily obtainable as well as easily compatible with usual food and/or feed ingredients. In addition the formulations, especially the solid formulations should be easily processible, e.g. provide low dusting properties, be easy dispersible or mixable in the matrix desired.

It is an object of the present invention to provide alternative stabilizing agents as well as to improve the stability, preferably thermo stability of phytases whereby stability is defined as the ability to retain activity under various conditions. This stability aspect relates to the entire life cycle of the enzyme, which comprises production (fermentation, downstream processing and formulation), distribution (transport and storage) and final application (production and storage of feed and/or food). For a commercially interesting enzyme, e.g. for phytase, it is important to withstand the high temperatures and high moisture reached during various feed and/or food treatment processes like pelleting, extrusion and expansion (up to 80-120°C) and to be stable during storage after addition to the feed and/or food, especially during long term storage. It is a further object of the invention to provide alternative stabilizers, which can be used in a smaller amount than those stabilizers known in the art, as the amount of stabilizer in the final formulations limits the further ingredients that can be added to an enzyme containing formulation. It is a further object of the invention to provide stabilizers that can be used especially for enzyme mixtures. If an enzyme preparation is prepared from more than one fermentation broth, the amount of stabilizer that can be added to the final formulation is limited. This is of special concern if a high enzyme concentration is desired in the final product and thus the amount of diluent that can be added to the final formulation is limited. In a further aspect of the invention, if an enzyme mixture is used, the stabilizer should also preferably stabilize not only one enzyme, but all enzymes in the mixture.

The term "stability" as used in the present invention relates to all specifications of an industrial enzyme, which comprise aspects such as activity, specificity, shelf-life stability, mechanical stability, microbial stability, toxicity, chemical composition and physical parameters such as density, viscosity, hygroscopy, but also colour, odour and dust. A preferred aspect of the present invention relates to the stability of an enzyme, preferably a phosphatase and/or a glycosidase against thermal inactivation during formulation and feed and/or food treatment processes such as pelleting, extrusion and expansion.

A major barrier to the wide use of phosphatases, especially phytases, is the constraint of thermal stability (80-120°C) required for these enzymes to withstand inactivation during feed and/or food treatment processes. Most of the currently available industrial phosphatases for feed and/or food applications have an insufficient intrinsic resistance to heat inactivation. As an alternative or in addition to molecular biological approaches the present invention enhances the stability, preferably thermostability of a phosphatase, especially phytase by the addition of different additives.

### Description of the Invention

In a first aspect of the present invention there is provided a stabilized solid or liquid enzyme formulation comprising at least one phytase and at least one stabilizing agent selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof, with the proviso that if gelatine is used in granules as solid formulations as the only stabilizing agent, the granules are subsequently coated.

The term "enzyme formulation" comprises all liquid and solid formulations in which the phytases may be commercialised. Preferably, the source of enzyme(s) for such a formulation is a rather raw, liquid preparation obtained from the fermentation broth. For the preparation of a liquid enzyme formulation according to the invention the selected stabilizing agents can be added directly to the fermentation broth or the fermentation broth can be purified, e.g. by filtration or ultrafiltration and the stabilising agent is then added after the filtration steps.

To obtain a stabilized, preferably thermo stabilized solid formulation the enzyme(s) can be spray-dried or granulated in the presence of the selected stabilizing agents.
A solid formulation is preferably a formulation, which contains less than 15 % (w/w), preferably less than 10 % (w/w), especially less than 8 % (w/w) of water.

In a preferred embodiment of the invention the stabilized enzyme formulation is solid, preferably in the form of a granule(s).

In a further, aspect of the present invention there is provided a process for the preparation of phytase-containing granule(s), the process comprising processing at least one phytase, a solid carrier which comprises at least 15% (w/w) of an edible car bohydrate polymer, and at least one stabilizing agent, wherein the stabilizing agent is selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof, with the proviso that if gelatine is used as the only stabilizing agent, the granules are subsequently coated. In a further aspect of the invention there is provided enzyme-containing granule(s) obtainable by such a process.

In a further aspect of the present invention there is provided a granule(s) comprising at least one phytase, a solid carrier which comprises at least 15% (w/w) of an edible carbohydrate polymer, and at least one stabilizing agent, wherein the stabilizing agent is selected from the group consisting agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof, with the proviso that if gelatine is used as the only stabilizing agent, the granules are subsequently coated.

In a further aspect of the present invention there is provided a granule(s) comprising at least one phytase, a solid carrier which comprises at least 15% (w/w) of an edible carbohydrate polymer, and at least one stabilizing agent, wherein the stabilizing agent is selected from the group consisting agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof, wherein the granule is coated.

The terms "granules" or "granule(s)" used throughout the description of the invention, both terms encompassing a single granule as well as a plurality of granules without distinction. The term granules and granulates are used synonymously.

The phytase-containing granule(s) obtainable by this process seeks to solve or at least mitigate the problems encountered in the prior art. The invention can thus provide processes for the preparation of stabilized enzyme formulations in the form of granulates that use the carbohydrate as a carrier. The carrier may be in particulate or powder form.

At least 15% (w/w) of the solid carrier is comprised of an edible carbohydrate polymer (such as starch). Preferably, however, at least 30% (w/w) of the solid carrier comprises the carbohydrate, optimally at least 40% (w/w). Advantageously the major component of the solid carrier is the carbohydrate (e.g. starch), for example more than 50% (w/w), preferably at least 60% (w/w), suitably at least 70% (w/w), and optimally at least 80% (w/w). These weight percentages are based on the total weight of the non-enzymatic components in the final dry granulate.

The edible carbohydrate polymer should be chosen so that it is edible by the animal or human for whom the feed or food, respectively is intended, and preferably at least partly digestible as well. The polymer preferably comprises glucose (e.g. a glucose-containing polymer), or (C₆H₁₀O₅)ₙ, units. Preferably the carbohydrate polymer comprises α-D-glucopyranose units, amylose (a linear (1->4) α-D-glucan polymer) and/or amylopectin (a branched D-glucan with α-D-(1->4) and α-D-(1->6) linkages). Starch is the preferred carbohydrate polymer. Other suitable glucose-containing polymers that can be used instead of, or in addition to starch, include α-glucans, β-glucans, pectin (such as proto-pectin), and glycogen. Derivatives of these carbohydrate polymers, such as ethers and/or esters thereof, are also contemplated although gelatinised starch is best avoided and thus may not be present. Suitably the carbohydrate polymer is water-insoluble.

Suitable carbohydrate polymers are com-, potato- and rice-starch. However, starch obtained from other (e.g. plant, such as vegetable or crop) sources such as tapioca, cassava, wheat, triticale, maize, sago, rye, oat, barley, yam, sorghum, or arrowroot is equally applicable. Similarly both native or modified (e.g. dextrin) types of starch can be used in the invention. Preferably the carbohydrate (e.g. starch) contains little or no protein, e.g. less than 5% (w/w), such as less than 2% (w/w) preferably less than 1 % (w/w). Regardless of the type of starch (or other carbohydrate polymer) it should be in a form that allows it to be used in an animal feed, in other words an edible or at least partly digestible form.

Water may be added to the processing. In a further embodiment of the invention, the granules are dried subsequent to the processing. It is understood that in one embodiment the granules can be dried irrespective of whether water was added to the processing or not.

The enzyme and water are preferably provided as an enzyme-containing (preferably aqueous) liquid, such as a solution or a slurry, which can be mixed with the solid carrier and allowed to absorb onto the carrier. During or after the mixing, the enzyme-containing liquid and the carrier are processed into a granule, which can then subsequently be dried. The use of the carbohydrate carrier may allow the absorption of large amounts of enzyme-containing liquid (and therefore enzyme). The mixture may be used to form a plastic paste or non-elastic dough that can readily be processed into granules, for example it can be extruded.

In the process of the invention the enzyme and water may be present in the same composition before contacting the solid carrier. In this respect, one may provide an enzyme-containing aqueous liquid. This liquid may be a solution or slurry that is from, or derived from, a fermentation process. This fermentation process will usually be one in which the enzyme is produced. The fermentation process may result in a broth that contains the microorganisms (which produce the desired enzyme) and an aqueous solution. This aqueous solution once separated from the micro organisms (for example, by filtration) can be the enzyme-containing aqueous liquid used in the invention. Thus in preferred embodiments the enzyme-containing aqueous liquid is a filtrate, especially a filtrate derived from a fermentation process resulting in production of enzyme. Depending on the process of separation that is used to isolate the enzyme from the fer mentation broth, the enzyme can also be present in a retentate.

The amount of enzyme-containing liquid (and so enzyme) that can be absorbed onto the carrier is usually limited by the amount of water that can be absorbed. Preferably the amount of liquid added to the solid carrier is such that (substantially) all the water in the (aqueous) liquid is absorbed by the carbohydrate present in the solid carrier.

At elevated temperatures starch and other carbohydrate polymers can absorb much larger amounts of water under swelling. For this reason the carbohydrate polymer is desirably able to absorb water (or enzyme-containing aqueous liquids). For example, corn starch can absorb up to three times its weight of water at 60°C and up to ten times at 70°C. The use of higher temperatures in order to absorb a greater amount enzyme-containing liquid is thus contemplated by the present invention, and indeed is preferable especially when dealing with thermostable enzymes. For these enzymes therefore the mixing of the solid carrier and liquid (or enzyme and water) and stabilizer can be conducted at elevated temperatures (e.g. above ambient temperature), such as above 30°C, preferably above 40°C and optimally above 50°C. Alternatively or in addition the liquid may be provided at this temperature.

However, in general, non-swelling conditions at lower (e.g. ambient) temperatures are preferred. This may minimise activity loss arising from instability of (heat sensitive) enzymes at higher temperatures. Suitably the temperature during the mixing of the enzyme and water is from 10 to 60°C, such as 10 to 50°C, preferably 20 to 40°C, preferably 20 to 25°C.

The mechanical processing used in the present invention for making the mixture of the enzyme, optionally water (e.g. an enzyme-containing liquid), the stabilizing agent and the solid carrier into granules (in other words granulating) can employ known techniques frequently used in food, feed and enzyme formulation processes. This may comprise expansion, extrusion, spheronisation, pelleting, high shear granulation, drum granulation, fluid bed agglomeration or a combination thereof. These processes are usually characterised by an input of mechanical energy, such as the drive of a screw, the rotation of a mixing mechanism, the pressure of a rolling mechanism of a pelleting apparatus, the movement of particles by a rotating bottom plate of a fluid bed agglomerator or the movement of the particles by a gas stream, or a combination thereof. These processes allow the solid carrier (e.g. in the form of a powder), to be mixed with the enzyme and optionally water, for example an enzyme-containing liquid (an aqueous solution or slurry), the stabilizer, and so subsequently granulated.

Alternatively the solid carrier can be mixed with the enzyme (e.g. in a powder form) and the stabilizing agent, to which optionally water, such as a liquid (or slurry) can then be added (which can act as granulating liquid).

In yet a further embodiment of the invention the granules (e.g. an agglomerate) is formed by spraying or coating the enzyme-containing liquid onto the carrier, which was previously mixed with the stabilizing agent, such as in a fluid bed agglomerator. Here the resulting granules can include an agglomerate as can be produced in a fluid bed agglomerator.

Preferably the mixing of the enzyme-containing liquid, the solid carrier and the stabilizing agent additionally comprises kneading of the mixture. This may improve the plasticity of the mixture in order to facilitate granulation (e.g. extrusion).

In a preferred embodiment the granulate is formed by extrusion, preferably by extrusion at low pressure. This may offer the advantage that the temperature of the mixture being extruded will not, or only slightly, increase. Low-pressure extrusion includes extrusion for example in a Fuji Paudal basket- or dome- extruder. The extrusion may naturally produce granules (the granules may break off after passage through a die) or a cutter may be employed.

Suitably the granules will have a water content of from 15 to 50%, such as 20 to 40%, such as from 25 to 35%, preferably 33 to 37% prior to drying. The enzyme content of the granules can be from 1 to 85 %, especially from 1 to 70%, such as 2 to 60%, preferably from 1 to 50%, preferably 2 to 25%, such as 3 to 15%, such as 5 to 12% (e.g. at least 50,000 ppm) prior to drying (always calculated as weight % based on the total weight of the granule).

The granules obtained can be subjected to rounding off (e.g. spheronisation), such as in a spheroniser, e.g. a MARUMERISER^{™} machine and/or compaction. If the obtained granules are dried, the spheronisation is preferably conducted prior to drying. The granules can be spheronised prior to drying since this may reduce dust formation in the final granulate and/or may facilitate any coating of the granulate.

The granules can then be dried, such as in a fluid bed drier or, in case of the fluid bed agglomeration, can be immediately dried (in the agglomerator) to obtain (solid dry) granules. Other known methods for drying granules in the food, feed or enzyme industry can be used by the skilled person. Suitably the granulate is flowable. The drying preferably takes place at a temperature of from 25 to 60°C, such as 30 to 50°C. Here the drying may last from 10 minutes to several hours. The length of time required will of course depend on the amount of granules to be dried.

After drying the granules, the resulting dried granules preferably have a water content of from 3 to 10%, such as from 5 to 9% by weight.

In a preferred embodiment of the invention there is provided a process wherein the process comprises:
a) mixing an aqueous liquid containing the enzyme with the solid carrier and the stabilizing agent;
b) mechanically processing the mixture obtained in a) to obtain enzyme-containing granules; and
c) drying the enzyme-containing granule(s) obtained in b).
wherein the stabilizing agent is selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof, with the proviso that if gelatine is used as the only stabilizing agent, the granules are subsequently coated.

In a further embodiment of the invention the granules are coated. A coating may be applied to the granule to give additional (e.g. favoured) characteristics or properties, like low dust content, colour, protection of the enzyme from the surrounding environment, different enzyme activities in one granulate or a combination thereof. The granules can be coated with or without prior drying. The granules can be coated with a fat, wax, polymer, salt, unguent and/or ointment or a coating (e.g. liquid) containing a (second) enzyme or a combination thereof. It will be apparent that if desired several layers of (different) coatings can be applied. To apply the coating(s) onto the granulates a number of known methods are available which include the use of a fluidised bed, a high shear granulator, a mixer granulator, or a Nauta-mixer.

In one embodiment the granules are coated, preferably after drying, for example to a residual moisture of less than about 10% by weight, with an organic polymer which is suitable for feedstuffs, by
(a) spraying the granules in a fluidized bed with a melt, a solution or a dispersion of the organic polymer or carrying out in a fluidized bed a powder coating with the organic polymer; or
(b) coating the granules in a mixer by melting on the organic polymer, or spraying the crude granulate with a melt, a solution or a dispersion of the organic polymer or carrying out a powder coating with the organic polymer;
and if necessary post-drying, cooling and/or freeing from coarse fractions the respective resultant polymer-coated granules.

According to a preferred embodiment of the process of the invention, the granules are charged into a fluidized bed, fluidized and coated with an aqueous or non-aqueous, preferably aqueous, solution or dispersion of the organic polymer by spraying. For this purpose a liquid which is as highly concentrated as possible and still spray able is used, for example an aqueous or non-aqueous solution or dispersion which contains from 10 to 50 weight-% of at least one polymer which is selected from the group consisting of
a) polyalkylene glycols, in particular polyethylene glycols having a number average molecular weight of from about 400 to 15,000, for example from about 400 to 10, 000;
b) polyalkylene oxide polymers or copolymers having a number average molecular weight of from about 4000 to 20,000, for example from about 7700 to 14,600; in particular block copolymers of polyoxyethylene and polyoxypropylene;
c) polyvinylpyrrolidone having a number average molecular weight from about 7000 to 1,000,000, for example from about 44,000 to 54,000
d) vinylpyrrolidone/vinylacetate copolymers having a number average molecular weight from about 30,000 to 100,000, for example from about 45,000 to 70,000;
e) polyvinyl alcohol having a number average molecular weight from about 10,000 to 200,000, for example from about 20,000 to 100,000; and
f) cellulose derivates, such as hydroxypropyl methyl cellulose having a number average molecular weight from about 6000 to 80,000, for example from about 12,000 to 65,000.

According to a further preferred process variant, for the coating a spray able aqueous or non-aqueous solution or dispersion is used, which contains from 10 to 40 weight-%, preferably from about 20 to 35 weight-% of at least one polymer which is selected from the group consisting of:
g) alkyl (meth)acrylate polymers and copolymers having a number average molecular weight from about 100,000 to 1,000,000; in particular ethyl acrylate/methyl methacrylate copolymers and methyl acrylate/ethyl acrylate copolymers; and
h) polyvinyl acetate having a number average molecular weight from about 250,000 to 700,000, possibly stabilized with polyvinylpyrrolidone is used.

Generally, preference is given to aqueous solutions or aqueous dispersions for the following reasons: No special measures are necessary for working up or recovering the solvents; some coating materials are preferentially offered as aqueous solutions or dispersions.

However, in special cases, the use of a non-aqueous solution or dispersion can also be advantageous. The coating material dissolves very readily or an advantageously high proportion of the coating material can be dispersed. In this manner a spray liquid having a high solids content can be sprayed, which leads to shorter process times. The lower enthalpy of evaporation of the non-aqueous solvent also leads to shorter process times.

Dispersions which can be used according to the invention are obtained by dispersing above polymers in an aqueous or non-aqueous, preferably aqueous, liquid phase, with or without a customary dispersant. A polymer solution or dispersion is preferably sprayed in such a manner that the granules are charged into a fluidized-bed apparatus or a mixer and the spray material is sprayed on with simultaneous heating of the charge. The energy is supplied in the fluidized-bed apparatus by contact with heated drying gas, frequently air, and in the mixer by contact with the heated wall and, if appropriate, with heated mixing tools. It may be expedient to preheat the solution or dispersion if as a result spray material can be sprayed with a high dry matter content. When organic liquid phases are used, solvent recovery is expedient. The product temperature during the coating should be in the range of from about 35 to 50°C. The coating can be carried out in the fluidized-bed apparatus in principle in the bottom-spray process (nozzle is in the gas-distributor plate and sprays upwards) or in the top-spray process (coating is sprayed from the top into the fluidized bed).

Examples of suitable polyalkylene glycols a) are: polypropylene glycols, and in particular polyethylene glycols of varying molar mass, for example PEG 4000 or PEG 6000, obtainable from BASF AG under the tradenames Lutrol E 4000 and Lutrol E 6000.

In a preferred embodiment the polyalkylene glycol coating is performed as described in WO 00/47060, page 4, finest to 14 and page 10, lines 28 to page 11, line 9, which is hereby incorporated by reference.

Examples of above polymers b) are: polyethylene oxides and polypropylene oxides, ethylene oxides/propylene oxide mixed polymers and block copolymers made up of polyethylene oxide and polypropylene oxide blocks, for example polymers which are obtainable from BASF AG under the tradenames Lutrol F 68 and Lutrol F127.
Of the polymers a) and b), preferably, highly concentrated solutions of from up to about 50% by weight, for example from about 30 to 50% by weight, based on the total weight of the solution, can advantageously be used.

Examples of above polymers c) are: polyvinylpyrrolidones, as are marketed, for example, by BASF AG under the tradenames Kollidon or Luviskol. Of these polymers, highly concentrated solutions having a solids content of from about 30 to 40% by weight, based on the total weight of the solution, can advantageously be used.

An example of abovementioned polymers d) is a vinylpyrrolidone/vinyl acetate copolymer which is marketed by BASF AG under the tradename Kollidon VA64. Highly concentrated solutions of from about 30 to 40% by weight, based on the total weight of the solution, of these copolymers can particularly advantageously be used.

Examples of above polymers e) are: products such as were marketed, for example, by Hoechst under the tradename Mowiol. Solutions of these polymers having a solids content in the range from about 8 to 20% by weight can advantageously be used.

Examples of suitable polymers f) are: hydroxypropylmethyl-celluloses, for example as marketed by Shin Etsu under the tradename Pharmacoat.

Examples of abovementioned polymers g) are: alkyl (meth)acrylate polymers and copolymers whose alkyl group has from 1 to 4 carbon atoms. Specific examples of suitable copolymers are: ethyl acrylate/methyl methacrylate copolymers, which are marketed, for example, under the tradenames Kollicoat EMM 30D by BASF AG or under the tradenames Eutragit NE 30 D by Röhm; also methacrylate/ethyl acrylate copolymers, as are marketed, for example, under the tradenames Kollicoat MAE 30DP by BASF AG or under the tradenames Eutragit 30/55 by Röhm. Copolymers of this type can be processed according to the invention, for example, as from 10 to 40 weight-% dispersions.

Examples of above polymers h) are: polyvinyl acetate dispersions which are stabilized with polyvinylpyrrolidone and are marketed, for example, under the tradename Kollicoat SR 30D by BASF AG (solids content of the dispersion from about 20 to 30% by weight).

According to a further preferred embodiment of the process of the invention, the granules are charged into a fluidized bed and powder-coated. The powder-coating is pref erably carried out using a powder of a solid polymer which is selected from the group consisting of hydroxypropyl methyl celluloses (HPMC) having a number average molecular weight of from about 6000 to 80,000; in a mixture with a plasticizer. Suitable materials for a powder coating are also all other coating materials which can be present in the pulverulent form and can be applied neither as a melt nor as highly concentrated solution (for example the case with HPMC).

The powder coating is preferably carried out in such a manner that the coating material is continuously added to the granules charged into the fluidized bed. The fine particles of the coating material (particle size in the range of from about 10 to 100,um) lie on the relatively rough surface of the crude granulate. By spraying in a plasticizer solution, the coating material particles are stuck together. Examples of suitable plasticizers are polyethylene glycol solutions, triethyl citrate, sorbitol solutions, paraffin oil and the like. To remove the solvent, the coating is performed with slight heating. The product temperature in this case is below about 60°C, for example from about 40 to 50°C.
In principle, the powder coating can also be carried out in a mixer. In this case, the powder mixture is added and the plasticizer is also injected via a nozzle. Drying is performed by supplying energy via the wall of the mixer and if appropriate via the mixing tools. Here also, as in the coating and drying in the fluidized bed, low product temperatures must be maintained.

According to a further preferred embodiment of the process of the invention, the granules are charged into a fluidized bed or mixer are coated using a melt of at least one polymer which is selected from the group consisting of
a) polyalkylene glycols, in particular polyethylene glycols, having a number average molecular weight of from about 1000 to 15,000; and
b) polyalkylene oxide polymers or copolymers having a number average molecular weight of from about 4000 to 20,000, in particular block copolymers of polyoxyethylene and polyoxypropylene.

The melt coating is carried out in a fluidized bed preferably in such a manner that the granulate to be coated is charged into the fluidized-bed apparatus. The coating material is melted in an external reservoir and pumped to the spray nozzle, for example, via a heatable line. Heating the nozzle gas is expedient. Spraying rate and melt inlet temperature must be set in such a manner that the coating material still runs readily on the surface of the granulate and coats this evenly. It is possible to preheat the granulate before the melts are sprayed. In the case of coating materials having a high melting point, attention must be paid to the fact that the product temperature must not be set too high in order to minimize loss of enzyme activity. The product temperature should be in the range of from about 35 to 50°C. The melt coating can also be carried out in principle by the bottom-spray process or by the top-spray process. The melt coating can be carried out in a mixer in two different ways. Either the granulate to be coated is charged into a suitable mixer and a melt of the coating material is sprayed into the mixer, or, in another possibility, the coating material in solid form is to be mixed with the product By supplying energy via the vessel wall or via the mixing tools, the coating material is melted and thus coats the crude granulate. If required, some release agent can be added from time to time. Suitable release agents are, for example, salicic acid, talcum, stearates and tricalcium phosphate.

The polymer solution, polymer dispersion or polymer melt used for the coating may receive other additions, for example of microcrystalline cellulose, talcum or kaolin.

In another embodiment of the invention the granules can be coated with a polyolefin as described in WO 03/059087, page 2, lines 19 to page 4, line 15.

In a further embodiment of the invention the granules can be coated with a dispersion comprising particle of a hydrophobic substance dispersed in a suitable solvent as described in WO 03/059086, page 2, line 18 to page 4 line 8. In a preferred embodiment of this coating, a polyolefin, especially preferred polyethylene and/or polypropylen are used.

Preferably the granules have a relatively narrow size distribution (e.g. they are monodisperse). This can facilitate a homogeneous distribution of the enzyme in the granules in the animal feed. The process of the invention tends to produce granulates with a narrow size distribution. However, if necessary, an additional step can be included in the process to further narrow the size distribution of the granules, such as screening. The mean particle size distribution of the granulate is suitably between 100 µm and 2000 µm, preferably between 200µm and 1800µm, preferably between 300µm and 1600 µm. The granules may be of irregular (but preferably regular) shape, for example approximately spherical. In a preferred embodiment the granules have a mean particle size distribution between 500 and 2000 µm, preferably between 500 and 1800µm, preferably between 600 and 1000 µm. The mean particle size distribution can be determined by using Mastersizer S, a machine of Malvem Instruments GmbH, Serial No., 32734-08. The mean particle size distribution is characterized by the values of D(v,0.1), D(v,0.5) and D(v,0.9) as well as the mean particle size of the distribution D(4,3).
The mean particle size distribution can be determined by sieving, e.g. with a sieving machine Typ Vibro VS 1000 distributed by Fa. Retsch.

A preferred process according to the invention therefore comprises:
a) mixing the (optional) water, enzyme, stabilizing agent and solid carrier comprising at least 15% (w/w) of an edible carbohydrate polymer, for example mixing the solid carrier and the at least one stabilizing agent with an aqueous enzyme-containing liquid;
b) optionally kneading the resulting mixture;
c) granulating, for example by mechanical processing, the mixture in order to obtain enzyme-containing granules, for example by using a granulator or by extrusion;
d) optionally spheronising the granules;
e) drying the resultant granules to obtain an enzyme-containing granulate.
wherein the stabilizing agent is selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal proteins and mixtures thereof, with the proviso that if gelatine is used as the only stabilizing agent, the granules are subsequently coated.

In a preferred embodiment the enzyme-containing granulated obtained in step e) is further coated.

In a preferred embodiment, one will aim to keep the maximum temperature to which the enzyme(s) are exposed to below 80°C during the entire process.

The water or enzyme-containing liquid may comprise one or more enzyme(s)in addition to the phytase. The enzymes (including the phytase) and are usually of microbial origin, e.g. obtained from a microbial fermentation. Usually the enzyme(s) will be in an active form (for example it may have catalytic or physiological activity). Preferably the liquid is in a concentrated form, such as an ultra-filtrate (UF) or a retentate, which may allow the production of a granulate with a desired activity level.

In a further aspect of the invention there is provided a liquid formulation comprising at least one phosphatase and at least one stabilizing agent, wherein the stabilizing agent is selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof.

The liquid formulation can be prepared using techniques commonly used in food, feed and enzyme formulation processes. In one embodiment, the stabilizing agent(s) can be added directly to the liquid in which the enzyme is solved or dispersed. In another embodiment of the invention the stabilizing agent(s) is first dissolved in additional water, optionally the pH of the obtained solution can be adjusted and the so obtained solution is subsequently mixed with the enzyme or enzyme concentrate or liquid enzyme preparation. A pH adjustment of the so obtained mixture is optional. The pH can be adjusted with organic or inorganic salts and/or acids.

In a preferred embodiment the liquid formulation comprises as at least one phosphatase a phytase.

The stabilizing agent is selected from the group consisting of consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof. It is understood that the stabilizing agent can be selected from one agent, e.g. only one gum, e.g. guaran or be composed of a mixture of e.g. one animal protein (e.g. albumin) and one gum (e.g. tragacanth) or a mixture of two or three or more different animal proteins or gums.

In a preferred embodiment the stabilizing agent is selected from agar, algin, carrageenan, furcelleran, ghatti gum, tragacanth gum, gum karya, guaran, locust bean gum (= carob bean gum), tamarind seed gum, arabinogalactan, xanthan (gum) or mixtures thereof.

In a preferred embodiment the stabilizing agent is selected from tragacanth gum, guaran, xanthan and/or locust bean gum.

In one embodiment the stabilizing agent is agar. Agar (or Agar-agar) is a heteropolysaccharide from red algae (Rhodophyceae). It is registered under the CAS no. 9002-18-0. Agar-agar is registered as a food additive under E 406.

In one embodiment the stabilizing agent is algin. The term "algin" encompasses alginic acid (CAS No.9005-32-7) and alginates, which are salts of alginic acid. Algin is a polysaccharide from brown algae (Phaeophyceae). Alginic acid is registered under E 400; sodium alginate is registered under the CAS No. 9005-38-5, E 401, Potassiumalginat as E 402, Ammoniumalginat as E 304, Calciumalginat as E 404 at propylenglykolalginate as E 405.

In one embodiment the stabilizing agent is carrageenan. Carrageenan is a polysaccharide from red algae (Rhodophyceae).

In one embodiment the stabilizing agent is furcellaran (= Danish agar). Furcellaran is a polysaccharide of the red algae Furcellaria fastigiata (CAS No. 9-000-21-9.

In one embodiment the stabilizing agent is ghatti gum. Ghatti gum is a polysaccharide from the exsudate of the bark of the tree Anogeissus latifolia (Combretaceae. It is registered under the CAS no. 9000-28-6.

In one embodiment the stabilizing agent is tragacanth gum. Tragacanth gum is the name for a exsudat from the stems and branches of bushes of the Astragalus-Family (Familie Fabaceae), especiall form Astralagus gummifer. It is registered under the CAS no. 9000-65-1, registered as a food additive under E.413.

In one embodiment the stabilizing agent is gum karya (= gum sterculia). Gum karya is the name for the exsudate of the tree Sterculia urens.

In one embodiment the stabilizing agent is guaran. Guaran is a polysaccharide from the guar bean (*Cyamopsis tetragonobolus*).

In one embodiment the stabilizing agent is locust bean gum (= carob bean gum, carubin). Locust bean gum is a polysaccharide from the seed of the locust bean trees. (*Ceratonia siliqua*) It is registered as food additive E 410.

In one embodiment the stabilizing agent is tamarind seed gum. Tamarind seed gum is a polysaccharid that is extracted from the grounded seed of the fruits of the tamarinde tree (Tamarindus indica L.).

In one embodiment the stabilizing agent is arabinogalactan. Arabinogalactan is a polysaccharide from L-Arabinose and D-Galactose. It is registered under CAS No. 9036-66-2.

In one embodiment the stabilizing agent is xanthan (gum). Xanthan is a microbial, anionic polysaccharide, which is excreted by *Xanthomonas campestris.* CAS No. 111 38-66-2. It is registered as a food additive E 415.

In one embodiment the stabilizing agent is at least one animal protein. In a preferred embodiment the stabilizing agent is at least one animal protein selected from the group consisting of proteins from poultry, beef, pig, fish and mixtures thereof.

An animal protein according to the invention can be obtained from any animal, e.g. from poultry, beef, pig or fish. Animals include non-ruminants or monogastric animals (pigs, fowl, poultry, marine animals such as fish), ruminants (bovine or ovine, e.g. cows, sheep, goats, deer, calves, lambs). Poultry includes chicken, hens, turkeys, ducks and geese.

The animal protein can be isolated from any part of the animal, e.g also from milk. As an animal protein will always be isolated from an animal, it can contain up to 20% (by weight), of non- protein components preferably up to 15%, preferably up to 10, especially up to 5%. An animal protein according to the invention can also be a complete or partial hydrolysate obtained from an animal protein.

In a preferred embodiment the animal protein is selected from the group consisting of gelatine, casein, albumin, and mixtures thereof.

Animal proteins suitable as stabilizers include, but are not limited to casein, albumin, milk protein, gelatine.

Gelatine (CAS No 9000-70-8, EINECS 232-554-6) can for example be obtained from Stoess under the name 100 BL A

Casein (CAS No. 900-71-9, EG-Nr. 232-555-1) can for example be obtained from Merck.

Albumin is a term that encompasses serum proteins from different sources. I includes lactalbumin (from milk), ovalbumin (from eggs) serum albumine, especially bovine serum albumin.

The stabilizing agent is usually added in an amount of 0.01 to 50 %, such as 1 to 30 % such as 1 to 20%, such as 3 to 10 weight-% based on the total weight of the mixture to be processed (for solid formulations) or based on the final liquid formulation. The stabilizing agent(s) can either be mixed with the carrier or with the enzyme or they can be added directly to the liquid formulations.

In other embodiments additional ingredients can be incorporated into the enzyme for mulation e.g. as processing aids, for further improvement of the pelleting stability and/or the storage stability. A number of such preferred additives are discussed below.

Salts may be included in the stabilized solid or liquid enzyme formulation, (e.g. with the solid carrier or water). Preferably (as suggested in EP-A-0,758,018) inorganic salt(s) can be added, which may improve the processing and storage stability. Preferred inorganic salts are water soluble. They may comprise a divalent cation, such as zinc (in particular), magnesium, and calcium or a monovalent cation such as sodium or potassium. Sulphate is the most favoured anion although other anions resulting in water solubility can be used (e.g. carbonate). The salts may be added (e.g. to the mixture) in solid form. However, the salt(s) can be dissolved in the water or enzyme-containing liquid prior to mixing with the solid carrier.

For liquid formulations the salt(s) can be added directly to the liquid enzyme formulations. Suitably the salt is provided at an amount that is from 0.1 to 40 weight-%, preferably 0.5 to 30, especially 1 to 25, preferably 1 to 20. (weight-% based on the final liquid formulation).

The solid formulation, e.g. granules, may therefore comprise less than 25% (w/w) of the salt, preferably less than 12%, for example from 2.5 to 7.5%, e.g. from 4 to 6%. (weight % based on the final solid formulation).

It is further contemplated that known stabilizing agent(s) can be added to the solid and/or liquid enzyme formulations, such as urea, glycerol, polyethylene glycol, preferably polyethylene glycol having a molecular weight of 6000 Da or mixtures thereof. Another example of further stabilizing agent(s) that can be added to the solid or liquid formulations are C5 sugars, preferably xylitol or ribitol, or sorbitol, polyethylene glycols having a molecular weight of 600 to 4000 Da, preferably 1000 to 3350 Da, the disodium salts of malonic, glutaric and succinic acid and carboxymethylcellulose.

Further improvement of the pelleting stability may be obtained by the incorporation of hydrophobic, gel-forming or slow dissolving (e.g. in water) compounds. These may be provided at from 1 to 10%, such as 2 to 8%, and preferably from 4 to 6% by weight (based on the weight of water and solid carrier ingredients for solid formulations) or 1 to 20 % based on the weight of the liquid formulation. Suitable substances include derivatised celluloses, such as HPMC (hydroxy-propyl-methyl-cellu lose), CMC (carboxy methyl-cellulose), HEC (hydroxy-ethyl-cellulose), microcristalline cellulose; polyvinyl alcohols (PVA); and/or edible oils. Edible oils, such as soy oil or canola oil, can be added (e.g. to the mixture to be granulated) as a processing aid.

The terms "enzyme" "enzyme(s)" and "enzymes" as used herein include single enzymes as well as mixtures of different enzymes (e.g. a phytase and an acid phosphatase as well as mixtures of the same enzyme of different origin (e.g. a fungal phytase and a bacterial phytase).

The enzyme formulations according to the invention comprise at least one phytase. Phytases encompass both 3-phytases and 6-phytases.

The term "phytase" means not only naturally occurring phytase enzymes, but any enzyme that possess phytase activity, for example the ability to catalyse the reaction involving the removal or liberation of inorganic phosphorus (phosphate) from myo-inositol phosphates. Preferably the phytase will belong to the class EC 3.1.3.8. The phytase can be a 3-phytase and/or a 6-phytase.

One unit of phytase activity (= FTU) is defined as the amount of enzyme which liberates 1 micromol of inorganic phosphorous per minute from 0.0051 mol/I of sodium phytate at ph 5.5 and 37 °C.

The analytical method is based on the liberation of inorganic phosphate from sodium phytate added in excess. The incubation time at pH 5.5 and 37 °C is 60 min. The phosphate liberated is determined via a yellow molybdenium-vanadium complex and evaluated photometrically at a wavelength of 415 nm. A phytase standard of known activity is run in parallel for comparison. The measured increase in absorbance on the product sample is expressed as a ratio to the standard (relative method, the official AOAC method).

The phytase activity can be determined according to "Determination of Phytase Activity in Feed by a Colorimetric Enzymatic Method": Collaborative Interlaboratory Study Engelen et al.: Journal of AOAC International Vol.84, No. 3, 2001.

The phytase according to the invention can be of microbial origin and/or it can be obtained by genetic modification of naturally occurring phytases and/or by de-novo construction (genetic engineering).

In a preferred embodiment the phytase is a plant phytase, a fungal phytase, a bacterial phytase or a phytase producible by a yeast.

Phytases are preferably derived from a microbial source such as bacteria, fungi and yeasts, but may also be of plant origin. In a preferred embodiment, the phytase is derived from a fungal strain, in particular a strain of Aspergillus, e.g. Aspergillus niger, Aspergillus oryzae, Aspergillus ficuum, Aspergillus awamori, Aspergillus fumigatus, Aspergillus nidulans and Aspergillus terreus. Most preferred is a phytase derived from a strain of Aspergillus niger or a strain of Aspergillus oryzae.

In another preferred embodiment, the phytase is derived from a bacterial strain, in particular a strain of Bacillus or a strain of Pseudomonas. Preferably the phytase enzyme is derived from a strain of Bacillus subtilis.

In another preferred embodiment, the phytase is derived from a bacterial strain, in particular a strain of E. coli.

In yet another preferred embodiment, the phytase is derived from a yeast, in particular a strain of Kluveromyces or a strain of Saccharomyces. Preferably the phytase is derived from a strain of Saccharomyces cerevisiae.

In the context of this Invention "an enzyme derived from" encompasses an enzyme naturally produced by the particular strain, either recovered from that strain or encoded by a DNA sequence isolated from this strain and produced in a host organism transformed with said DNA sequence.

The phytase may be derived from the microorganism in question by use of any suitable technique. In particular, the phytase enzyme may be obtained by fermentation of a phytase-producing microorganism in a suitable nutrient medium, followed by isolation of the enzyme by methods known in the art.

The broth or medium used for culturing may be any conventional medium suitable for growing the host cell in question, and may be composed according to the principles of the prior art. The medium preferably contains carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains.

After cultivation, the phytase enzyme is recovered by conventional method for isolation and purification proteins from a culture broth. Well known purification procedures include separating the cells from the medium by centrifugation or filtration, precipitating proteinaceous components of the medium by means of a salt such as ammonium sulphate, and chromatographic methods such as e.g. ion exchange chromatography, gel filtration chromatography, affinity chromatography, etc.

Alternatively, the phytase enzyme is preferably produced in larger quantities using recombinant DNA techniques, e.g. as described in EP-A1 -0 420 358, which publication is hereby incorporated by reference.

Preferably, a fungus of the species Aspergillus which has been transformed with the phytase-encoding gene obtained from the species Aspergillus ficuum or Aspergillus niger, is cultured under conditions conducive to the expression of the phytase-encoding gene as described in EP-A1-0 420 358.

The phytase-containing fermentation broth is preferably treated by means of both filtration and ultra-filtration prior to being used in the formulation of the present invention.

In a further preferred embodiment of the invention, phytases derived by molecular engineering are used, e.g. genetically modified phytases as described in WO 94/03072 (Röhm), in WO 99/49022 (Novozymes), in WO 00/43503 (Novozymes) or in WO 03/102174 (BASF AG).

Another phytase preferably used in this invention is the so-called consensus phytase. This is a phytase developed according to a theoretical molecular biological approach, which has a higher intrinsic stability compared with Aspergillus phytases, see European Patent Application Publication No. 897 985. In the practice of the present invention the consensus phytases specifically described in examples 3-13 can also be used.

It is also possible to produce such phytases by genetic engineering whereby the gene obtained from a fungus is transferred to a host organism like a bacterium (e.g. E. coli), a yeast or another fungus, for further details, see e.g. European Patent Application Publication No. 68431 3 and European Patent Application Publication No. 897 010.

In a preferred embodiment of the present invention a phytase according to EP-B1 420 358 can be used.

In a preferred embodiment of the invention the enzyme formulation of the invention further comprises additional enzyme(s).

Preferred additional enzymes for the formulations of the present invention include those enzymes useful in food (including baking) and feed industries.

Such enzymes include but are not limited to proteases (bacterial, fungal, acid, neutral or alkaline), preferably with a neutral and/or acidic pH optimum.

The protease (proteolytic enzyme) may be a microbial enzyme, preferably a protease derived from a bacterial or a fungal strain or the protease may be trypsin or pepsin. In a preferred embodiment, the proteolytic enzyme is a bacterial protease derived from a strain of Bacillus, preferably a strain of Bacillus subtilis or a strain of Bacillus licheniformis. Commercially available Bacillus proteases are Alcase^{™} and Neutrase^{™} (Novozymes, Denmark). In another preferred embodiment, the proteolytic enzyme is a fungal protease derived from a strain of Aspergillus, preferably a strain of Aspergillus aculeatus, a strain of Aspergillus niger, a strain of Aspergillus oryzae. A commercially available Aspergillus protease is Flavourzyme^{™} (Novozymes, Denmark).

Such enzymes include but are not limited to lipases (fungal, bacterial, mammalian), preferably phospholipases such as the mammalian pancreatic phospholipases A2 or any triacylglycerol lipase (E.C. 3.1.1.3).

Such enzymes include but are not limited to glucose oxidases.

Such enzymes include but are not limited to glycosidases (E.C. 3.2, also know as carbohydrases), e.g. amylases (alpha or beta), cellulases (whole cellulase or functional components thereof), in particular xylanases, glucanases, preferably β-glucanases, galactosidases, pectinases, and galactosidases, such as α- or β-galctosidases.

The glycosidase enzyme may be any glycosidase enzyme (EC 3.2.1, also known as carbohydrases). Preferably, the glycosidase enzyme is an amylase, in particular an α-amylase or a β-amylase, a cellulase, a glucanase, in particular an endo-1,4-β-glucanase (E.C. 3.2.1.4) or an endo-1,3-β-glucanase (E.C. 3.2.1.6), a xylanase, in par ticular an endo-1,4-β-xylanase (E.C. 3.2.1.8) or a xylan-endo-1,3-β-xylosidase (E.C. 3.2.1.32), an α-galactosidase (E.C. 3.2.1.22), a polygalacturonase (E.C.3.2.1.15, also known as pectinase), a cellulose-1,4-β-cellobiosidase (E.C. 3.2.1.91, also known as cellobiohydrolases), an endo-glucanase, in particular an endo-1,6-δ-glucanase (E.C. 3.2.1.75), an endo-1,2-β-glucanase (E.C. 3.2.1.71), an endo-1,3-β-gtucanase (E.C. 3.2.1.39) or an endo-1,3-α-glucanase (E.C. 3.2.1.59). They also include mannanases, such as β-*Mannosidase* (EC 3.2.1.25) and *Mannan-Endo-1,4-*β*-Mannosidase* (EC 3.2.1.78).

A preferred endo-1,4-β-glucanase (E.C. 3.2.1.4) according to this invention is the endo-1,4-β-glucanase described in WO 01/70998 (BASF AG), which is hereby incorporated by reference.

In a preferred embodiment the additional enzyme is selected from the group consisting of glycosidases, phospholipases and glucose oxidases.

In a preferred embodiment the additional enzyme is selected from the group consisting of glycosidases.

In a preferred embodiment the additional enzyme is selected from the group consisting of xylanases, glucanases and mixtures thereof.

In a preferred embodiment of the invention the additional enzyme is at least one xylanase. Xylanases can be obtained from microbial source, e.g. such as Aspergillus niger, Clostridium thermocellum, Trichoderma reesei, Penicillium janthinellum, as well as species of Bacillus and Streptomyces. The xylanase can also be obtained by recombinant expression e.g. as described in EP 121 138. In a preferred embodiment a xylanase as described in EP 0 463 706 B1 (BASF AG) and/or in WO 02/24926 A1 (BASF AG) can used according to the invention.

Xylanases suitable according to the invention can be endo-xylanases and/or exo-xylanases, preferably endo-xylanases, especially endo-β-xylanases.

Suitable additional enzyme(s) are those to be included in animal feed which includes pet food and/or in human nutrition. The function of these enzymes is often to improve the feed conversion rate, e.g. by reducing the viscosity or by reducing the anti-nutritional effect of certain feed compounds. Feed enzymes can also be used, such as to reduce the amount of compounds in the manure which are harmful to the environment.

When the enzyme formulations of the present invention are to be used in food applications, the enzyme(s) must be food quality.

It is within the scope of the invention that at least one, preferably two, preferably three or more different enzymes are used. These can be enzymes from the same class, e.g. two different phytases or enzymes from different classes, e.g. a phytase and a xylanase. It is to be understood that whenever referred to the enzyme or an enzyme, also mixtures of enzymes are included in these terms, irrespective of whether such mixtures are obtainable directly in a single fermentation or by mixing enzymes obtainable in different fermentations; and further including enzymes obtainable by fermentation of recombinant organisms.

If the phosphatase is a phytase, then preferably the solid or liquid enzyme formulation will have an activity of from 1,000 to 80,000, such as 2,000 to 70,000, such as 3,000 to 60,000, preferably 4,000 to 50,000, such as 5,000 to 25,000, such as from 5,000 to 15,000, such as 5,000 to 10,000 such as from 6,000 to 8,000, FTU/g.

If the additional enzyme is a plant cell wall degrading enzyme, such as xylanase, then the final solid or liquid formulation may have an activity of the enzyme ranging from 3,000 to 500,000, preferably 4,000 to 250,000, preferably 5,000 to 100,000, preferably 6,000 to 80,000, and optimally 8,000 to 70,000, EXU/g.

If the additional enzyme is a cellulase, such as a glucanase, preferably a J3-glucanase, then the solid or liquid enzyme formulation can have an enzyme activity of from 100 to 150,000, such as 500 to 100,000, preferably 750 to 50,000, preferably from 1,000 to 10,000, and optimally from 1,500 to 7,000, BGU/g.

One endo-xylanase activity (EXU) is defined as the amount of enzyme which liberates 1.00 micromole reducing sugars, measured as xylose equivalents, per minute under the conditions of the test (pH 3,5 and 55°C).

One beta-glucanase unit (BGU) is defined as the amount of enzyme which liberates 0.258 micromole reducing sugars expressed as glucose equivalents per minute under the conditions of the test (pH 3,5 and 40°C).

The endo-xylanase activity (EXU) and the β-glucanase activity (BGU) can be determined according to Engelen et al: Journal of AOAC International Vol. 79, No. 5, 1019 (1996).

The solid formulations, e.g. the granules may comprise from 1 to 20, e.g. from 5 to 20, e.g. from 7 to 15 weight% of the enzyme(s) (phosphatase plus additional enzymes).

The liquid formulations may comprise from 0.5 to 20, e.g. from 5 to 15 weight % of the enzyme(s) (phosphatase plus additional enzymes).

A further aspect of the present invention relates to a process for the preparation of animal feed, or a premix or precursor to an animal feed, the process comprising mixing a solid and/or liquid enzyme formulation as defined in any of the claim 1 to 10 and/or claim 26 with one or more animal feed substances (e.g. seeds) or ingredients. This can then be sterilised, e.g. subjected to heat treatment. The resulting composition can then suitably be processed into pellets and optionally dried.

The enzyme supplemented feed can be subjected to several methods of feed processing like extrusion, expansion and pelleting, where temporarily high temperatures may occure and thermostabilisation is an advantage.

The stabilized enzyme formulation of the present invention can be applied for example on feed pellets. The thermo-stabilised liquid enzyme formulation may be diluted with tap water to yield a solution having the desired activity of the enzyme. In case the phosphatase is phytase, the solution is preferably diluted so that an activity of 100 to 1000, preferably 200 to 700 FTU/g, especially 300 to 500 F1'U/g solution is obtained. The feed pellets can be transferred to a mechanical mixer and the diluted enzyme formulation is sprayed onto the feed pellets while being agitated in order to yield a homogeneous product with an added enzyme activity. Examples for phytase containing feed pellets will preferably result in activities of about 500 FTU/kg feed pellets.

Alternatively the solid or liquid enzyme formulation can be directly mixed with the mash feed before this mixture is then subjected to a process such as pelleting, expansion or extrusion.

A further aspect of the present invention relates to a process for the preparation of a composition, or a premix or a precursor suitable for human nutrition, the process comprising mixing a solid and/or liquid enzyme formulation as defined in any of the claim 1 to 10 and/or claim 26 with one or more food substance(s) or ingredient(s). This can then be sterilised, e.g. subjected to heat treatment. The resulting composition can then suitably be processed into pellets and optionally dried.

Preferably the solid formulations (e.g. granules) and feed or food substance(s) or ingredient(s) are mixed in a ration of granules: feed or food substance(s) or ingredients which is at lower than 1 g:1 kg, preferably lower than 0.5 g : 1 kg, especially lower than 0.1 g : 1 kg, preferably lower than 0.05 g : 1 kg.

A further aspect of the invention relates to the use of a solid and/or liquid enzyme formulation as defined in any of the claim 1 to 10 and/or claim 26 for human and/or animal nutrition. The invention therefore encompasses compositions such as animal feed compositions or compositions suitable for human nutrition. These compositions are preferably in the form of pellets (there may be 1-5, e.g. 2-4 dried granules per pellet). These composition can have a water content of from 8 to 20%, e.g. from 12-15%. The amount of enzyme(s) can be from 0.0001 to 0.005 weight-%, suitably from 0.0005 to 0.0012 weight-%, such as at least 5 ppm.

In a further aspect the present invention concerns a method-of providing a monogastric animal with its dietary requirement of phosphorus wherein the animal is fed with a feed according to the present invention and whereby no additional phosphate is added to the feed.

A further aspect relates to a process for promoting the growth of an animal and/or improvement of the feed conversion rate, the process comprising feeding an animal with a diet that comprises a solid and/or liquid enzyme formulation as defined in any of the claim 1 to 10 and/or claim 26.

Suitably the composition comprises from 0.05 to 2.0, such as 0.3 to 1.0, optimally 0.4 to 0.6 FTU/g of a phosphatase, e.g. a phytase. A xylanase may be present from 0.5 to 50, e.g. 1 to 40 EXU/g, preferably 2 to 25, especially 2 to 15 EXU/g. Alternatively or in addition a cellulase may be present from 0.05 to 1.5, e.g. 0.1 to 1,0, e.g. 0.2 to 0.6 BGU/g.

A further aspect of the present invention relates to the use of a solid and/or liquid enzyme formulation as defined in any of the claim 1 to 10 and/or claim 26 in, or as a component of, an animal feed or for use in an animal diet.

The solid and/or liquid enzyme formulation as defined in any of the claim 1 to 10 and/or claim 26, when fed to animal and/or human, contribute to the reduction of environmental pollution. They furthermore, when fed to animals improve the quality of the animal product (e.g. meat, eggs) obtained from an animal fed with a solid and/or liquid enzyme formulation as defined in any of the claim 1 to 10 and/or claim 26.

Suitable animals include farm animals (pigs, poultry, livestock), non-ruminants or monogastric animals (pigs, fowl, poultry, marine animals such as fish), ruminants (bovine or ovine, e.g. cows, sheep, goats, deer, calves, lambs). Poultry includes chicken, hens, turkeys, geese and ducks.
A further aspect of the invention relates to the use of at least one stabilizing agent selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof in a phosphatase containing composition to improve the pelleting stability of the enzyme.

A further aspect of the invention relates to the use of at least one stabilizing agent selected from the group consisting of agar, algin, carrageenan, furcelleran, ghatti gum, tragacanth gum, gum karya, guaran, locust bean gum (= carob bean gum), tamarind seed gum, arabinogalactan, xanthan (gum), at least one animal protein and mixtures thereof as additives for the production of solid and/or liquid enzyme formulations, preferably for solid enzyme formulations. In this embodiment of the present invention the stabilizing agent(s) are preferably added as solid compounds to a standard granulation mixture. Such formulation can result in an increased recovery (up to 20%) of e.g. phytase activity determined after a high shear granulation process which included a drying step of the granulates on a fluid bed dryer at 45°C for 15 min. In addition such granulates which contain the stabilizing agent(s) according to the invention can show, when mixed with feed and/or food, an increased recovery of enzymatic activity after the feed and/or food treatment (e.g. a pelleting process at 85°C) compared to granulates without such additives.

Preferred features and characteristics of one aspect of the invention are equally applicable to another *mutatis mutandis.*

### Examples

All percentages are (w/w) unless otherwise specified.

### EXAMPLE V 1 (Comparison)

Preparation of corn starch-based enzyme granules by kneading, extrusion, spheronisation and drying

In a first step corn starch was filled into a laboratory mixer (Loedige Typ M5 RMK) and slowly agitated. A mixture of an ultra-filtrate containing phytase, ZnSO₄ x 6 H₂O, PVA (polyvinyl alcohol) and additional water was poured into a beaker and dissolved/dispersed. This mixture was poured onto the corn starch in the Loedige mixer.

Thus, an enzyme containing dough was obtained by mixing and kneading 66% (w/w) of corn starch, 20% (w/w) of an ultra-filtrate containing phytase, 1 % (w/w) of PVA, 0,3% (w/w) of ZnSO₄ x 6 H₂O and 12,7% (w/w) of additional water at 100 - 250 rpm in the Loedige mixer.

In a second step this enzyme containing dough was extruded using an Fuji Paudal laboratory extruder, type DG-L1, to obtain a wet extrudate which was then rounded in a laboratory spheroniser to obtain round particles of an average diameter of 500 - 800 µm.

These particles were subsequently dried in a fluid bed dried (Niro-Aeromatic, type MP-1) for 60 - 90 minutes at a bed temperature of approximately 40°C. Approximately 1,5 kg of the wet granules were dried and subsequently cooled down. The obtained dry enzyme granules had an activity of 8920 FTU/g.

| Example V1 | corn starch | Ultrafiltrate | PVA | ZnSO₄ x 6 H₂O | water |
|---|---|---|---|---|---|
| dough | 66,0 % | 20.0% | 1,0 % | 0,3 % | 12,7% |
| granules | 83,6 % | 8,6 % | 1,4 % | 0,4 % | 6,0 % |

### EXAMPLE 1: casein as stabilizer

In a first step corn starch and casein (obtained from Merck, Darmstadt, CAS No. 900-71-9 was filled into a laboratory mixer (Loedige Typ M5 RMK) and slowly agitated. A mixture of an ultra-filtrate containing phytase, ZnSO₄ x 6 H₂O and additional water was poured into a beaker and dissolved/dispersed. This mixture was poured onto the corn starch and the casein in the Loedige mixer. Thus, a phytase containing dough was obtained by mixing and kneading the ingredients at 100 - 250 rpm in the Loedige mixer.

As described in example V1 enzyme containing granules were obtained by extruding the dough, spheronising, drying and cooling. The obtained dry enzyme granules had an activity of 7690 FTU/g.

| Example 1 | corn starch | ultrafiltrate | Casein | ZnSO₄ x 6 H₂O | water |
|---|---|---|---|---|---|
| dough | 62,0 % | 20,0 % | 5,0 % | 0,3 % | 12,7% |
| granules | 78,6 % | 8,5 % | 6,5 % | 0,4 % | 6,0 % |

### EXAMPLE 2: casein as stabilizer

Enzyme (phytase) containing granules were obtained according to example 1. The obtained dry enzyme granules had an activity of 7580 FTU/g.

| Example 2 | corn starch | ultrafiltrate | casein | ZnSO₄ x 6 H₂O | water |
|---|---|---|---|---|---|
| dough | 47,0 % | 20,0 % | 20,0 % | 0,3 % | 12,7% |
| granules | 59,6 % | 8,6 % | 25,4 % | 0,4 % | 6,0 % |

### Comparison of pelleting stabilities

The phytase granules of the invention were subjected to a pelleting trial and their pelleting stability was compared to granules made according to the standard recipe (= Example V1. The pelleting trial consisted of mixing the different enzyme granules with a standard broiler diet at a level of 1 g/kg feed. This enzyme containing feed was treated by injection of steam in a conditioner and immediately afterwards pelleted in a pelleting press to obtain feed pellets, which were subsequently cooled. Pellet temperature directly measured after pelleting press was 78°C. This type of process is typically used in feed industry to obtain feed pellets. Example 1 as well as example 2 showed an increase of at least 10% in pelleting stability as compared to example V1.

## Claims

1. A stabilized solid or liquid enzyme formulation comprising at least one phytase and at least one stabilizing agent, wherein the stabilizing agent is at least one stabilizing animal protein, with the proviso that if gelatine is used in granules as solid formulations as the only stabilizing agent, the granules are subsequently coated.

2. Enzyme formulation according to claim 1, wherein the phytase is a plant phytase, a fungal phytase, a bacterial phytase, a phytase producible by a yeast or a consensus phytase.

3. Enzyme formulation according to any preceding claim, wherein the animal protein is selected from the group consisting of proteins from poultry, beef, pig, fish and mixtures thereof.

4. Enzyme formulation according to any preceding claim, wherein the animal protein is selected from the group consisting of gelatine, casein, albumin and mixtures thereof.

5. Enzyme formulation according to any preceding Claim comprising at least one additional stabilizing agent selected from the group consisting of agar, algin, carrageenan, furcelleran, ghatti gum, tragacanth gum, gum karya, guaran, locust bean gum (= carob bean gum), tamarind seed gum, arabinogalactan and xanthan (gum).

6. Enzyme formulation according to any claim 1 to 5 **characterized in that** the formulation is liquid.

7. Enzyme formulation according to any claim 1 to 5, **characterized in that** the formulation is solid.

8. Enzyme formulation according to claim 7, **characterized in that** the solid formulation is in the form of granule(s).

9. Enzyme formulation according to claim 8, wherein the granule(s) comprise a solid carrier which comprises at least 15 % (w/w) of an edible carbohydrate polymer.

10. Enzyme formulation according to claim 9, wherein the granule(s) is coated.

11. A process for the preparation of phytase-containing granule(s), the process comprising processing
(i) at least one phytase,
(ii) a solid carrier which comprises at least 15 % (w/w) of an edible carbohydrate polymer, and
(iii) at least one stabilizing agent, wherein the stabilizing agent is at least one animal protein, with the proviso that if gelatine is used as the only stabilizing agent, the granules are subsequently coated.

12. A process according to claim 11 wherein water is added to the processing.

13. A process according to any claim 11 to 12 wherein the water and phytase are provided as enzyme-containing aqueous liquid(s).

14. A process according to claim 13 wherein the liquid is a filtrate derived from a fermentation process resulting in production of the phytase.

15. A process according to any claim 11 to 14 wherein the granules are dried subsequent to the processing.

16. A process according to any claim 11 to 15 wherein the animal protein is selected from the group consisting of proteins from poultry, beef, pig, fish and mixtures thereof.

17. A process according to any claim 11 to 16 wherein the animal protein is selected from the group consisting of gelatine, casein, albumin and mixtures thereof.

18. A process according to any claim 11 to 17 wherein the stabilizing agent comprises at least one additional stabilizing agent selected from the group consisting of agar, algin, carrageenan, furcelleran, ghatti gum, tragacanth gum, gum karya, guaran, locust bean gum (= carob bean gum), tamarind seed gum, arabinogalactan and xanthan (gum).

19. A process according to any claim 11 to 18 wherein the process comprises:
a) mixing an aqueous liquid containing the phytase with the solid carrier and the stabilizing agent;
b) mechanically processing the mixture obtained in a) to obtain phytasecontaining granules; and
c) drying the phytase-containing granule(s) obtained in b).

20. A process according to any claim 11 to 19 wherein the processing is mechanical and comprises extrusion, pelleting, high-shear granulation, expansion, fluid bed agglomeration, spheronisation, drum granulation or a combination thereof.

21. A process according to any claim 11 to 20 wherein the phytase-containing aqueous liquid, the solid carrier and the stabilizing agent are mixed and the resuming mixture is kneaded before granulation.

22. A process according to any claim 11 to 21 wherein the processing is extrusion performed at low pressure and/or in a basket- or dome-extruder.

23. A process according to any claim 11 to 22 wherein the granule(s) are spheronised.

24. A process according to any claim 11 to 23 wherein the granule(s) are coated.

25. A process according to any claim 11 to 24 wherein the phytase is a plant phytase, a fungal phytase, a bacterial phytase, a phytase producible by a yeast or a consensus phytase.

26. A process according to any claim 11 to 25, wherein the granule(s) will have phytase activity ranging from 1,000 to 80,000 FTU/g, preferably from 2,000 to 70,000 FTU/g, preferably 3,000 to 60,000 FTU/g, more preferably 4,000 to 50,000 FTU/g and more preferably from 5,000 to 15,000 FTU/g.

27. Phytase-containing granule(s) obtainable by a process as defined in any claim 11 to 26.

28. A process for the preparation of an animal feed, or a premix or precursor to an animal feed, the process comprising mixing a stabilized solid and/or liquid formulation according to any claim 1 to 10 and/or claim 27 with one or more animal feed substance(s) or ingredient(s).

29. A process for the preparation of a composition, or a premix or a precursor suitable for human nutrition, the process comprising mixing a stabilized solid and/or liquid formulation according to any claim 1 to 10 and/or claim 27 with one or more food substance(s) or ingredient(s).

30. A process according to any claim 28 to 29 wherein the mixture of feed or food substance(s) and stabilized solid and/or liquid formulation according to any claim 1 to 10 and/or claim 27 is sterilised or treated with steam, pelletised and optionally dried.

31. Use of stabilized solid and/or liquid formulation according to any claim 1 to 10 and/or claim 27 for human and/or animal nutrition.

32. A process for promoting the growth of an animal and/or improving the feed conversion rate, the process comprising feeding an animal with a diet that comprises stabilized solid and/or liquid formulation according to any claim 1 to 10 and/or claim 27.

## Patentansprüche

1. Stabilisierte feste oder flüssige Enzymformulierung, die mindestens eine Phytase und mindestens ein Stabilisierungsmittel enthält, wobei es sich bei dem Stabilisierungsmittel um mindestens ein stabilisierendes tierisches Protein handelt, mit der Maßgabe, daß, wenn Gelatine in Granulatkörnern als einziges Stabilisierungsmittel verwendet wird, die Granulatkörner anschließend beschichtet werden.

2. Enzymformulierung nach Anspruch 1, wobei es sich bei der Phytase um eine pflanzliche Phytase, eine pilzliche Phytase, eine bakterielle Phytase, eine von einer Hefe erhältliche Phytase oder eine Consensus-Phytase handelt.

3. Enzymformulierung nach einem der vorhergehenden Ansprüche, wobei das tierische Protein aus der Gruppe der Geflügel-, Rinder-, Schweine-, Fischproteine und Mischungen davon stammt.

4. Enzymformulierung nach einem der vorhergehenden Ansprüche, wobei das tierische Protein aus der Gruppe Gelatine, Casein, Albumin und Mischungen davon stammt.

5. Enzymformulierung nach einem der vorhergehenden Ansprüche, die mindestens ein zusätzliches Stabilisierungsmittel aus der Gruppe Agar-Agar, Algin, Carrageen, Furcellaran, Ghatti-Gummi, Traganth-Gummi, Karya-Gummi, Guaran, Johannisbrotkernmehl, Tamarindensamengummi, Arabinogalactan und Xanthan(yGummi) umfaßt.

6. Enzymformulierung nach einem der Ansprüche 1 bis 5, **dadurch gekennzeichnet, daß** die Formulierung flüssig ist.

7. Enzymformulierung nach einem der Ansprüche 1 bis 5, **dadurch gekennzeichnet, daß** die Formulierung fest ist.

8. Enzymformulierung nach Anspruch 7, **dadurch gekennzeichnet, daß** die feste Formulierung in Form von Granulat (-körnern) vorliegt.

9. Enzymformulierung nach Anspruch 8, wobei das Granulat (bzw. die Granulatkörner) einen festen Träger umfaßt/umfassen können, der mindestens 15% (w/w) eines genießbaren Kohlenhydratpolymers umfaßt.

10. Enzymformulierung nach Anspruch 9, wobei das Granulat (bzw. die Granulatkörner) beschichtet ist/sind.

11. Verfahren zur Herstellung eines phytasehaltigen Granulats bzw. von phytasehaltigen Granulatkörnern, wobei das Verfahren die Verarbeitung von
(i) mindestens einer Phytase,
(ii) einem festen Träger, der mindestens 15% (w/w) eines genießbaren Kohlenhydratpolymers umfaßt, und
(iii) mindestens einem Stabilisierungsmittel, wobei es sich bei dem Stabilisierungsmittel um mindestens ein stabilisierendes tierisches Protein handelt, mit der Maßgabe, daß, wenn Gelatine als einziges Stabilisierungsmittel verwendet wird, die Granulatkörner anschließend beschichtet werden, umfaßt.

12. Verfahren nach Anspruch 11, wobei Wasser zum Verarbeiten zugegeben wird.

13. Verfahren nach Anspruch 11 oder 12, wobei das Wasser und die Phytase als enzymhaltige wäßrige Flüssigkeit(en) bereitgestellt werden.

14. Verfahren nach Anspruch 13, wobei es sich bei der Flüssigkeit um ein Filtrat handelt, das von einem Fermentationsvorgang, der zur Produktion der Phytase führt, stammt.

15. Verfahren nach einem der Ansprüche 11 bis 14, wobei das Granulat nach der Verarbeitung getrocknet wird.

16. Verfahren nach einem der Ansprüche 11 bis 15, wobei das tierische Protein aus der Gruppe der Geflügel-, Rinder-, Schweine-, Fischproteine und Mischungen davon stammt.

17. Verfahren nach einem der Ansprüche 11 bis 16, wobei das tierische Protein aus der Gruppe Gelatine, Casein, Albumin und Mischungen davon stammt.

18. Verfahren nach einem der Ansprüche 11 bis 17, wobei das Stabilisierungsmittel mindestens ein zusätzliches Stabilisierungsmittel aus der Gruppe Agar-Agar, Algin, Carrageen, Furcellaran, Ghatti-Gummi, Traganth-Gummi, Karya-Gummi, Guaran, Johannisbrotkernmehl, Tamarindensamengummi, Arabinogalactan und Xanthan(-Gummi) umfaßt.

19. Verfahren nach einem der Ansprüche 11 bis 18, das folgendes umfaßt:
a) Mischen einer wäßrigen Flüssigkeit, die die Phytase enthält, mit dem festen Träger und dem Stabilisierungsmittel;
b) mechanisches Verarbeiten der in a) erhaltenen Mischung, wodurch man phytasehaltige Granulatkörner erhält; und
c) Trocknen des/der in b) erhaltenen phytasehaltigen Granulats/Granulatkörner.

20. Verfahren nach einem der Ansprüche 11 bis 19, wobei es sich bei dem Verarbeiten um ein mechanisches Verarbeiten handelt, das Extrudieren, Pelletieren, Granulieren unter hochscherenden Bedingungen, Expandieren, Wirbelschichtagglomeration oder eine Kombination davon umfaßt.

21. Verfahren nach einem der Ansprüche 11 bis 20, wobei die phytasehaltige wäßrige Flüssigkeit, der feste Träger und das Stabilisierungsmittel vermischt werden und die erhaltene Mischung vor dem Granulieren geknetet wird.

22. Verfahren nach einem der Ansprüche 11 bis 21, wobei es sich bei dem Verarbeiten um Extrudieren unter niedrigem Druck und/oder im Korb- oder Kuppelextruder handelt.

23. Verfahren nach einem der Ansprüche 11 bis 22, wobei das Granulat bzw. die Granulatkörner sphäronisiert werden.

24. Verfahren nach einem der Ansprüche 11 bis 23, wobei das Granulat bzw. die Granulatkörner beschichtet werden.

25. Verfahren nach einem der Ansprüche 11 bis 24, wobei es sich bei der Phytase um eine pflanzliche Phytase, eine pilzliche Phytase, eine bakterielle Phytase, eine von einer Hefe erhältliche Phytase oder eine Consensus-Phytase handelt.

26. Verfahren nach einem der Ansprüche 11 bis 25, wobei das Granulat bzw. die Granulatkörner eine Phytaseaktivität im Bereich von 1.000 bis 80.000 FTU/g, vorzugsweise 2.000 bis 70.000 FTU/g, vorzugsweise 3.000 bis 60.000 FTU/g, stärker bevorzugt 4.000 bis 50.000 FTU/g und stärker bevorzugt 5.000 bis 15.000 FTU/g aufweist/aufweisen.

27. Phytasehaitige(s) Granulat/Granulatkörner, erhältlich nach einem Verfahren gemäß einem der Ansprüche 11 bis 26.

28. Verfahren zur Herstellung eines Tierfutters bzw. einer Vormischung oder Vorstufe für ein Tierfutter, wobei das Verfahren umfaßt, daß man eine stabilisierte feste und/oder flüssige Formulierung nach einem der Ansprüche 1 bis 10 und/oder Anspruch 27 mit einem oder mehreren Tierfutterstoff(en) oder -bestandteil(en) vermischt.

29. Verfahren zur Herstellung einer Zusammensetzung oder einer Vormischung oder Vorstufe für eine Zusammensetzung, wobei das Verfahren umfaßt, daß man eine stabilisierte feste und/oder flüssige Formulierung nach einem der Ansprüche 1 bis 10 und/oder Anspruch 27 mit einem oder mehreren Tierfutterstoff(en) oder -bestandteil(en) vermischt.

30. Verfahren nach Anspruch 28 oder 29, wobei die Mischung aus Futterstoff(en) oder Futtersubstanz(en) und stabilisierter fester und/oder flüssiger Formulierung nach einem der Ansprüche 1 bis 10 und/oder Anspruch 27 sterilisiert oder mit Dampf behandelt, pelletiert und gegebenenfalls getrocknet wird.

31. Verwendung einer stabilisierten festen und/oder flüssigen Formulierung nach einem der Ansprüche 1 bis 10 und/oder Anspruch 27 für die Ernährung von Mensch und/oder Tier.

32. Verfahren zur Förderung des Wachstums eines Tiers und/oder zur Verbesserung der Futterumsatzrate, wobei man bei dem Verfahren dem Tier eine Ration verfüttert, die eine wäßrige und/oder flüssige Formulierung nach einem der Ansprüche 1 bis 10 und/oder Anspruch 27 umfaßt.

## Revendications

1. Formulation enzymatique stabilisée, solide ou liquide, comprenant au moins une phytase et au moins un agent stabilisant, dans laquelle l'agent stabilisant est au moins une protéine animale stabilisante, à condition que lorsque la gélatine est utilisée comme unique agent stabilisant dans les granulés en tant que formulations solides, les granulés en question soient ensuite enrobés.

2. Formulation enzymatique selon la revendication 1, dans laquelle la phytase est une phytase végétale, une phytase fongique, une phytase bactérienne, une phytase pouvant être produite par une levure ou une phytase de consensus.

3. Formulation enzymatique selon l'une quelconque des revendications précédentes, dans laquelle la protéine animale est choisie dans le groupe constitué des protéines de volaille, de boeuf, de porc, de poisson et de leurs mélanges.

4. Formulation enzymatique selon l'une quelconque des revendications précédentes, dans laquelle la protéine animale est choisie dans le groupe constitué de la gélatine, de la caséine, de l'albumine et de leurs mélanges.

5. Formulation enzymatique selon l'une quelconque des revendications précédentes, comprenant au moins un agent stabilisant supplémentaire choisi dans le groupe constitué de l'agar, de l'algine, de la carraghénine, du furcellarane, de la gomme ghatti, de la gomme de tragacanthe, de la gomme de karaya, de la gomme de guar, de la gomme de caroube (= gomme de fève de caroube), de la gomme de graines de tamarin, d'un arabinogalactane et du xanthane (gomme).

6. Formulation enzymatique selon l'une quelconque des revendications 1 à 5, **caractérisée en ce que** la formulation est liquide.

7. Formulation enzymatique selon l'une quelconque des revendications 1 à 5, **caractérisée en ce que** la formulation est solide.

8. Formulation enzymatique selon la revendication 7, **caractérisée en ce que** la formulation solide se présente sous la forme de granulés.

9. Formulation enzymatique selon la revendication 8, dans laquelle les granulés comprennent un véhicule solide, qui est constitué d'au moins 15 % (p/p) d'un polymère carbohydraté comestible.

10. Formulation enzymatique selon la revendication 9, dans laquelle les granulés sont enrobés.

11. Procédé de préparation de granulés à teneur en phytase, le procédé consistant à traiter :
(i) au moins une phytase,
(ii) un véhicule solide qui est constitué d'au moins 15 % (p/p) d'un polymère carbohydraté comestible, et
(iii) au moins un agent stabilisant, dans lequel l'agent stabilisant est au moins une protéine animale et à condition que les granulés soient enrobés si la gélatine est utilisée comme unique agent stabilisant.

12. Procédé selon la revendication 11, dans lequel on ajoute de l'eau lors du traitement.

13. Procédé selon l'une quelconque des revendications 11 à 12, dans lequel l'eau et la phytase sont fournies sous la forme d'un ou de plusieurs liquides aqueux contenant l'enzyme.

14. Procédé selon la revendication 13, dans lequel le liquide est un filtrat dérivé d'un procédé de fermentation aboutissant à la production de la phytase.

15. Procédé selon l'une quelconque des revendications 11 à 14, dans lequel les granulés sont séchés après le traitement.

16. Procédé selon l'une quelconque des revendications 11 à 15, dans lequel la protéine animale est choisie dans le groupe constitué des protéines de volaille, de boeuf, de porc, de poisson et de leurs mélanges.

17. Procédé selon l'une quelconque des revendications 11 à 16, dans lequel la protéine animale est choisie dans le groupe constitué de la gélatine, de la caséine, de l'albumine et de leurs mélanges.

18. Procédé selon l'une quelconque des revendications 11 à 17, dans lequel l'agent stabilisant comprend au moins un agent stabilisant supplémentaire choisi dans le groupe constitué de l'agar, de l'algine, du carraghénane, du furcellarane, de la gomme ghatti, de la gomme de tragacanthe, de la gomme de karaya, de la gomme de guar, de la gomme de caroube (= gomme de fève de caroube), de la gomme de graines de tamarin, d'un arabinogalactane et du xanthane (gomme).

19. Procédé selon l'une quelconque des revendications 11 à 18, le procédé en question comprenant les étapes consistant à :
a) mélanger un liquide aqueux contenant la phytase au véhicule solide et à l'agent stabilisant ;
b) soumettre le mélange obtenu au point a) à un traitement mécanique de manière à obtenir des granulés contenant la phytase ; et
c) sécher les granulés contenant la phytase obtenus au point b).

20. Procédé selon l'une quelconque des revendications 11 à 19, dans lequel le traitement est mécanique et comprend l'extrusion, le pastillage, la granulation à taux de cisaillement élevé, l'expansion, l'agglomération en lit fluidisé, la sphéronisation, la granulation en tambour ou leur combinaison.

21. Procédé selon l'une quelconque des revendications 11 à 20, dans lequel on mélange le liquide aqueux contenant la phytase, le véhicule solide et l'agent stabilisant, et on malaxe le mélange obtenu avant d'effectuer la granulation.

22. Procédé selon l'une quelconque des revendications 11 à 21, dans lequel le traitement est une extrusion effectuée à faible pression et/ou réalisée dans une extrudeuse à panier ou à dôme.

23. Procédé selon l'une quelconque des revendications 11 à 22, dans lequel les granulés sont sphéronisés.

24. Procédé selon l'une quelconque des revendications 11 à 23, dans lequel les granulés sont enrobés.

25. Procédé selon l'une quelconque des revendications 11 à 24, dans lequel la phytase est une phytase végétale, une phytase fongique, une phytase bactérienne, une phytase pouvant être produite par une levure ou une phytase de consensus.

26. Procédé selon l'une quelconque des revendications 11 à 25, dans lequel les granulés présenteront une activité de phytase située dans la plage de 1000 à 80 000 FTU/g, de préférence de 2000 à 70 000 FTU/g, mieux encore de 3000 à 60 000 FTU/g, bien mieux encore de 4000 à 50 000 FTU/g et, nettement mieux encore de 5000 à 15 000 FTU/g.

27. Granulés à teneur en phytase pouvant être obtenus par un procédé comme défini selon l'une quelconque des revendications 11 à 26.

28. Procédé pour préparer un aliment pour animaux ou un prémélange ou un précurseur d'aliment pour animaux, le procédé comprenant le mélange d'une formulation stabilisée solide et/ou liquide selon l'une quelconque des revendications 1 à 10 et/ou selon la revendication 27, à une ou plusieurs substances ou ingrédients d'aliments pour animaux.

29. Procédé pour préparer une composition ou un prémélange ou un précurseur convenant à l'alimentation humaine, le procédé comprenant le mélange d'une formulation stabilisée solide et/ou liquide selon l'une quelconque des revendications 1 à 10 et/ou selon la revendication 27 à une ou plusieurs substances ou ingrédients alimentaires.

30. Procédé selon l'une quelconque des revendications 28 et 29, dans lequel le mélange de substances destinées à l'alimentation humaine ou animale et de la formulation stabilisée, solide et/ou liquide, selon l'une quelconque des revendications 1 à 10 et/ou selon la revendication 27, est/ou soumis à une stérilisation, est soumis à un traitement à la vapeur d'eau, à un pastillage et, éventuellement, à un séchage.

31. Utilisation de la formulation stabilisée, solide et/ou liquide, selon l'une quelconque des revendications 1 à 10 et/ou selon la revendication 27, pour l'alimentation humaine et/ou animale.

32. Procédé destiné à favoriser la croissance d'un animal et/ou à améliorer le taux de conversion des aliments, le procédé comprenant l'alimentation d'un animal selon un régime qui comprend une formulation stabilisée, solide et/ou liquide, selon l'une quelconque des revendications 1 à 10 et/ou selon la revendication 27.
